# EUROPEAN PATENT SPECIFICATION

(11) **EP 0 324 725 B1**
(45) Date of publication and mention of the grant of the patent: **31.03.1993**
(21) Application number: 89850006.1
(22) Date of filing: 12.01.1989
(51) Int. Cl.: A61K 9/18, A61K 47/00, A61K 9/14

(54) **A pharmaceutical composition**
Pharmazeutische Zubereitung
Préparation pharmaceutique

(30) Priority: 13.01.1988 SE 8800080
(43) Date of publication of application: 19.07.1989
(73) Proprietor: Kabi Pharmacia AB, S-751 82 Uppsala (SE)
(72) Inventor: Nyström, Christer, S-752 51 Uppsala (SE); Westerberg, Marie, S-172 34 Sundbyberg (SE); Nilsson, Per, S-151 52 Södertälje (SE)
(74) Representative: Onn, Thorsten

(56) References cited:
- SE-A- 8 600 657
- JOURNAL OF PHARM. PHARMACOL., vol. 39, February 1987, pages 769-773; H. TAKEUCHI et al.: "Enhancement of the dissolution rate of a poorly water-soluble drug by a spray-drying solvent deposition and desintegrants".
- DRUG DEV. IND. PHARM. vol. 11, no. 4, 1985, pages 891-900; J. MC.GINNITY et al.: "Dissolution properties of ordered mixtures of griseofulvin and a directly compressible excipient".
- INTERNATIONAL JOURNAL OF PHARMACEUTICALS, vol. 28, August 1985, pages 23-31; M.WESTERBERG et al.: "Physicochemical aspects of drug release".
- POWDER TECHNOLOGY, ed. by K. Iinoya et al., Hemisphere publishing corp. 1984, pages 734-41; J. MC.GINITY et al.: "Ordered mixtures of hydrophobic drugs and a directly compressible excipient".

## Description

The present invention relates generally to the field of drug formulation, and more specifically, but not exclusively, to ordered solid mixtures of particulate pharmaceutical carrier substances and smaller particles of at least one pharmaceutically active substance or agent, said small particles adhering to the carrier substance particles. The invention also relates to pharmaceutical preparations produced from the ordered mixtures and to a method for producing such preparations. Furthermore, the invention also relates to the use of the ordered mixtures for the preparation of said pharmaceutical preparations.

It is known that ordered mixtures of a relatively coarse particulate, water-soluble, pharmaceutically acceptable carrier substance and smaller particles of at least one pharmaceutically active substance can be produced by means of a dry mixing process, in which the smaller particles adhere or bind to the surfaces of the larger carrier particles (C. Nyström et al., J. Pharm. Pharmacol. 38, pages 161-165, (1986); M. Westerberg et al, International Journal of Pharmaceutics, 28, pages 23-31, (1986)). Because of their small particle size, the therapeutically active substances will normally form agglomerates which render the formulations not-readily soluble in water. In the case of formulations produced by the aforesaid dry mixing processes, however, the active substance(s) is (are) distributed uniformly over the surfaces of readily dissolvable carrier particles, therewith enabling the therapeutically active substances to dissolve more quickly. Consequently, the active substances will dissolve more rapidly when administered and consequently act more quickly, in a highly advantageous manner.

These mixtures, however, have a significant limitation. When an attempt is made to increase the amount of pharmaceutically active substance contained by the mixture, it is found that the speed at which the formulations dissolve will decrease rapidly when a certain proportion is exceeded, therewith losing all of the advantages earlier afforded. This phenomenon creates a serious drawback when desiring to administer pharmaceutical preparations which contain high proportions of active substance(s).

The so-called surface area ratio, Rₛ, constitutes a measurement of the quantity of particulate therapeutically active agent which will adhere to the surfaces of the larger carrier particles. Rₛ is defined as the ratio of the projected surface of the adherent particles to the total external surface area of the carrier particles. A more detailed description of how this surface area ratio is determined is given by C. Nyström, J. Mazur and J. Sjögren in International Journal of Pharmaceutics, 10: pages 209-218 (1982). It is found that the dissolution rate decreases markedly at an Rₛ-value as low as about 0.5 and that at values approaching or exceeding Rₛ = 1, the dissolution rate has decreased to such an extent that none of the aforesaid advantages are attained. This is assumed to be because at higher Rₛ-values the adherent particles form on the surface of the carrier particles a layer which prevents rapid penetration of the water phase to the underlying carrier substance and subsequent instantaneous dissolution thereof. These circumstances represent a return to the earlier circumstances in which the particles of therapeutically active substance(s) bake together to form agglomerates, in the absence of a carrier.

These disadvantages are eliminated by the present invention, with which there is obtained an ordered mixture in accordance with the aforegoing which will dissolve rapidly in water, despite containing high proportions of adherent particles of therapeutically active substance(s) and consequently having a high Rₛ-value.

This effect is achieved in accordance with the invention, in that the carrier substance particles have a pharmaceutical disintegrant (or "explosive") embodied therein. This disintegrant is effective in causing the carrier particles to disintegrate and dissolve rapidly when coming into contact with water, so as to release the individual, mutually discrete primary particles of active substance.

In the accompanying drawings, Figure 1 is a diagram which illustrates the dependency of the dissolution rate on the various values of the surface area ratio. Figure 2 illustrates a comparison made between the dissolution rates of earlier known mixtures and those of mixtures according to the present invention. Figure 3 shows the result of a further comparison test and is illustrative of the significance of the nature of the carrier.

The carrier particles will suitably contain up to 25% by weight disintegrant, preferably up to 10% by weight. In practice, contents below 1% by weight have been found to give an excessively low effect and an optimum content would appear to be from about 5 to 10% percent.

A suitable carrier particle size is from 50 to 1000 µm, and then preferably from 100 to 500 µm. Although particle sizes which lie outside the widest range can also be used, practical difficulties are experienced when formulating pharmaceutical preparations from particles having such sizes.

The carrier substance used may be any substance which is acceptable pharmaceutically and which is highly soluble in water and can be formed into particles which embody or incorporate a disintegrant. A number of such substances are known to the person skilled in this art, of which carbohydrates, such as mannitol and lactose, or pharmaceutically acceptable inorganic salts, such as sodium chloride, can be mentioned as examples. In accordance with one particularly preferred embodiment of the invention, the carrier substances comprises a fragmentizing material. By a fragmentizing material is meant a material which is readily crushed or broken up when a pharmaceutical composition is compacted into tablets, so as to expose further surfaces. The aforesaid mannitol and lactose have been found particularly suitable in this respect.

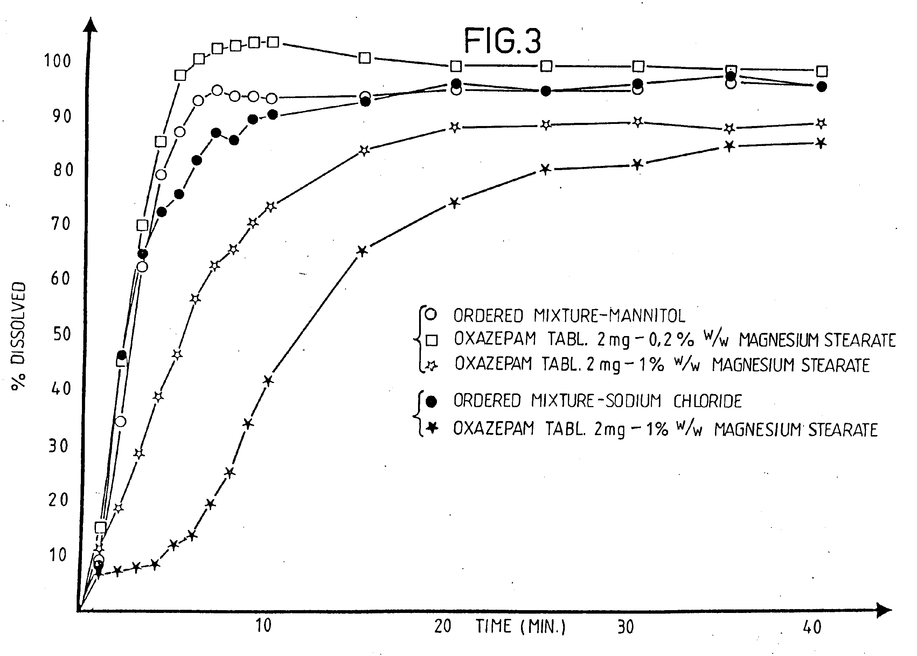

As will be evident from Figure 3, the use of a fragmentizing material as a carrier substance has been found particularly necessary when the ordered mixture includes a lubricant.

The pharmaceutical disintegrant may comprise any substance which is known for this purpose by those skilled in this art. Particularly effective agents in this respect are those which swell drastically in water, through hydratization, and thus exhibit an increase in volume of up to 10-20 times their dry volume. Examples of such agents are cellulose and starch derivatives in the form of water-insoluble, cross-linked polymers which swell markedly in water. Derivatives of polyvinylpyrrolidone are other examples of such agents. One particularly suitable disintegrant for use in accordance with the present invention is a modified cellulose gum which is highly swellable in water and which is retailed under the trade name Ac-Di-Sol^{R} by FMC Corporation, USA. Other types of disintegrant can also be used, and the person skilled in this art will have no trouble in choosing a suitable agent for the purpose intended.

The disintegrant can be embodied in the carrier particles in various ways. For example, the finely divided carrier can be granulated together with finely divided disintegrant in a liquid which will not dissolve the disintegrant or cause it to swell. In this case, the dry constituents are first mixed together and the resultant mix is then moistened with a liquid, such as absolute ethanol, which will not dissolve the substances present or cause swelling. The mass is then granulated, for instance by pressing the moist mass through a filter cloth and then drying and grinding the mass. Alternatively, the moist mass can be dried immediately and then granulated. Another way is to dissolve the carrier substance in a solvent which will not dissolve the disintegrant or cause it to swell, and then to add the disintegrant to the solution. The mixture is then evaporated and the dry residue subsequently granulated. Other methods are also conceivable to the person skilled in the art. Irrespective of the method applied, a suitable grain size fraction of carrier with disintegrant shall be prepared in a final stage, e.g. by passing the granulate through an appropriate screen or sieve.

Examples of different kinds of active substance which have been found useable in accordance with the invention include benzodiazepines, ergotamine tartrate, isosorbide dinitrate and griseofulvin. This list of active substances is by no means complete, however, since there can be used any agent which can be finely divided and caused to adhere in finely divided form to the surfaces of the carrier particles. The advantages afforded by the invention are achieved primarily with the use of active substances which are not dissolved readily in water and with which the degree of solubility can be varied in dependence on the active substance used. It will be understood that the degree of solubility of the active substances places no limitation on the scope of the invention.

Several mutually different pharmaceutically active substances can be applied simultaneously to (mutually) the same carrier particles.

The pharmaceutically active substances present in the preparations will suitably have a maximum particle size of about 24 µm, preferably not greater than about 10 µm.

The active substances are caused to adhere to the carrier particles by dry mixing the ingredients together over a sufficiently long period of time. This time period can vary and when mixing the ingredients on a laboratory scale can be of the order of from 10 to 100 hours, depending specifically on the substances used and the details of the mixing process applied. When mixing is effected on a larger scale, and then particularly on an industrial scale, the requisite mixing time will be much shorter, normally in the order of from 10 to 60 minutes. One of normal skill in this art will have no difficulty in determining a suitable mixing time for a given combination of active substances and carrier on the basis of simple routine experiments.

In accordance with one preferred embodiment of the invention it has been found suitable when mixing the active substance and carrier particles together, to add also a proportion of pure carrier particles whose particle size is of the same order as that of the active substance. This will further enhance the subsequent dissolution of the substance in water. This proportion of pure, finely divided carrier added during the mixing process may equal 100% by weight of the amount of active substance present, and is suitably about 40% by weight. This additional carrier may comprise the same substance as that of the carrier which embodies the disintegrant, or may comprise another pharmaceutically acceptable carrier substance.

The value obtained with the surface area ratio defined in the aforegoing is significant with respect to the amount of pharmaceutically active substance which shall adhere to the surfaces of the carrier particles. In the case of the inventive preparations, the surface area ratio will preferably not be greater than about 0.5. A ratio of this value will afford significant improvements with respect to dissolution rate in comparison with similar preparations which have the same surface area ratio, but with which the carrier particles lack the inclusion of a disintegrant. Improvements on preparations which lack the inclusion of a disintegrant in the carrier particles, but which are similar in other respects, will also be achieved at higher surface area ratio values. The improvements achieved in this latter case, however, are not so pronounced in the former case and are therefore of less practical importance.

The ordered mixtures prepared in accordance with the invention can be incorporated in various kinds of pharmaceutical preparations. Such preparations are preferably intended for enteral administration, primarily orally. The preparations will then, for instance, be in the form of tables, capsules, powders or granulates, or in the form of suppositories for rectal administration. It is also possible to use the ordered mixtures of the present invention in certain preparations or medical materia intended for external application, such as ointments or creams. Irrespective of the form given to the preparation, it is important that the preparation is essentially free from water, since the presence of water would result in premature dissolution of the active substance.

The pharmaceutical preparations can be composed, by combining the inventive ordered mixtures with the conventional pharmaceutical additives and excipients normally used in the desired forms of the preparations, with the aid of known methods herefor. Such additions may comprise, for instance, additional carriers, preservatives, lubricants, glidants, disintegrants, flavourants, dyestuffs and like substances, and are well known to the person skilled in this art.

It is important in this context to distinguish between the earlier known use of pharmaceutical disintegrants and the use of such substances in accordance with the present invention. In accordance with the known use, the disintegrant shall lie in the tablet between the mutually compacted carrier particles, active substance particles and particles of other substances present and contributes towards rapid disintegration of the tablet when the tablet is administered or taken. In distinction, the disintegrant used in accordance with the present invention is embodied within the carrier particles and assists in causing the particles to dissolve rapidly. However, there is nothing to prevent both of these disintegrant functions being utilized in one and the same tablet, such that a final tablet composition will include both carrier particles which embody a disintegrant there-within and which have smaller particles of active substance adhering to the surfaces thereof and also a further disintegrant which is intended to exercise its conventional function.

The invention is illustrated in more detail in the following with reference to non-limiting examples and with reference to the accompanying drawings.

### Example 1

This example illustrates the effect of the surface area ratio on the rate of dissolution when using carrier particles which do not have a disintegrant embodied therein.

Dry mannitol having a particle size of 250-450 µm was mixed dry with micronized oxazepam, the degree of fineness being such as to obtain an external specific surface area of not less than 20000 cm²/g, measured in accordance with a permeametric surface method. Surface area ratios of 0.08, 0.57 and 1.5 were obtained, corresponding to an oxazepam addition of 0.4% w/w, 2% w/w and 5% w/w respectively. The mixing time was about 50 hours. Each of the three ordered mixtures thus obtained was then divided into three parts. The dissolution rate of each of the mixtures having mutually different surface area ratio values was then investigated directly in accordance with U.S.P. XXI, pages 1243-1244, the paddle method, at room temperature and at a stirring or paddle speed of 100 rpm.

The two remaining parts of each respective mixture were formulated into tablets, by admixing the ordered mixture with 5% w/w Avicel^{R}, PH 101 (trade name for microcrystalline cellulose) and 2% w/w Ac-Di-Sol ^{R} (trade name for a modified cellulose gum of high water-swellability) over a time period of 30 minutes, and then compacting the resultant mixtures into tablets containing 2 mg oxazepam in the case of mixtures having a surface area ratio of 0.08 and 10 mg oxazepam in the case of mixtures having surface area ratios of 0.57 and 1.5 respectively. The mixtures were compacted at two mutually different pressures of 100 MPa and 200 MPa. The dissolution rate of these tablets was similarly investigated in accordance with U.S.P. XXI, the paddle method, at room temperature and a stirring speed of 100 rpm.

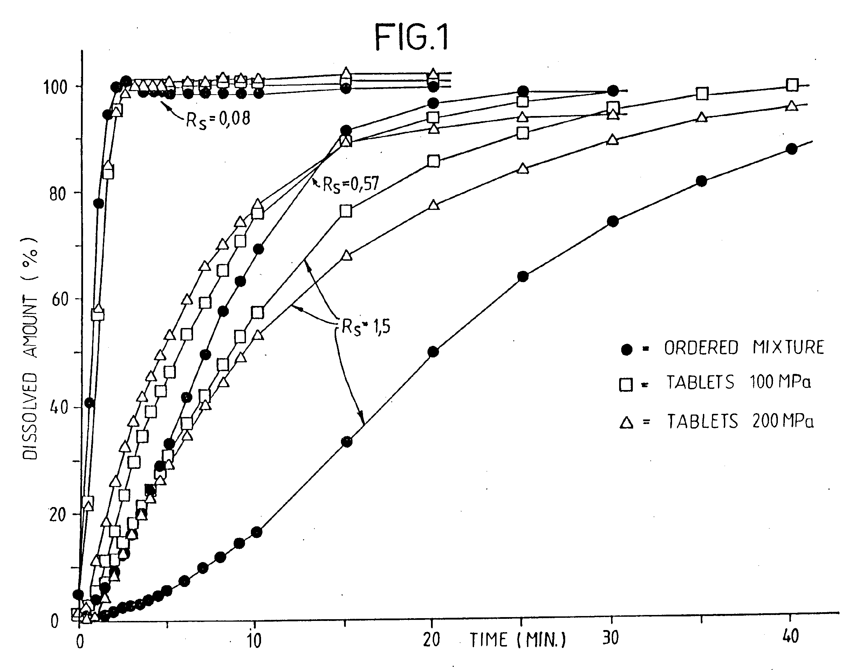

The results obtained are illustrated by the curves presented in Figure 1. These curves show clearly that in the case of a surface area ratio of 0.08, the mixture or tablets dissolved very quickly, at a rate which scarcely varied between the ordered mixture as such and the tablets produced from this mixture.

The curves also show that the dissolution rate decreased markedly in the case of mixtures having a surface area ratio of 0.57, and that a time period of about 30 minutes was needed to obtain near completion dissolution, as compared with a time period of about 2 minutes for the mixtures which had a surface area ratio of 0.08. The curves also show that initially the tablets dissolve more rapidly than the mixture alone.

In the case of a surface area ratio of 1.5, which thus corresponds to a carrier particle coating comprising more than one single particle layer of the active substance, the dissolution rate has decreased so markedly as to render the mixture unusable in practice for the formulation of pharmaceutical preparations, which are required to have a rapid onset. It is evident from the curves that the tablets dissolve much more quickly than the ordered mixture alone. This is assumed to be because carrier particles are fragmented when the mixture is compacted into tablet form, such as to expose uncoated surfaces to the dissolving effect of the water present. The non-fragmented particles, on the other hand, are covered completely with a coating of not-readily dissolved active substance, which prevents the access of water to the soluble carrier.

### Example 2

This example illustrates the preparation of an ordered mixture in accordance with the invention and its formulation into tablets.

A batch of 100 tablets was produced from the following compositions: 36.5 g of mannitol and 1.92 g of Ac-Di-Sol ^{R} were mixed with about 170 ml of absolute ethanol. The mixture was dried and forced through a sieve of 1 mm mesh width, and the resultant fraction of particle size between 250 and 450 µm was mixed with 500 mg of micronized oxazepam, which had a specific surface area of not less than 20000 cm²/g and with 0.1 g of finely ground mannitol, over a period of 50 hrs. The amount of oxazepam included corresponded to a surface area ratio of 0.5. This mixture was then admixed with 2.00 g of Avicel^{R} PH 101 and 0.78 g of Ac-Di-Sol ^{R}, the ingredients being thoroughly mixed together over a period of 60 minutes. The mixture was then compacted into tablets at a compacting pressure of 200 MPa, each tablet containing 5 mg of oxazepam.

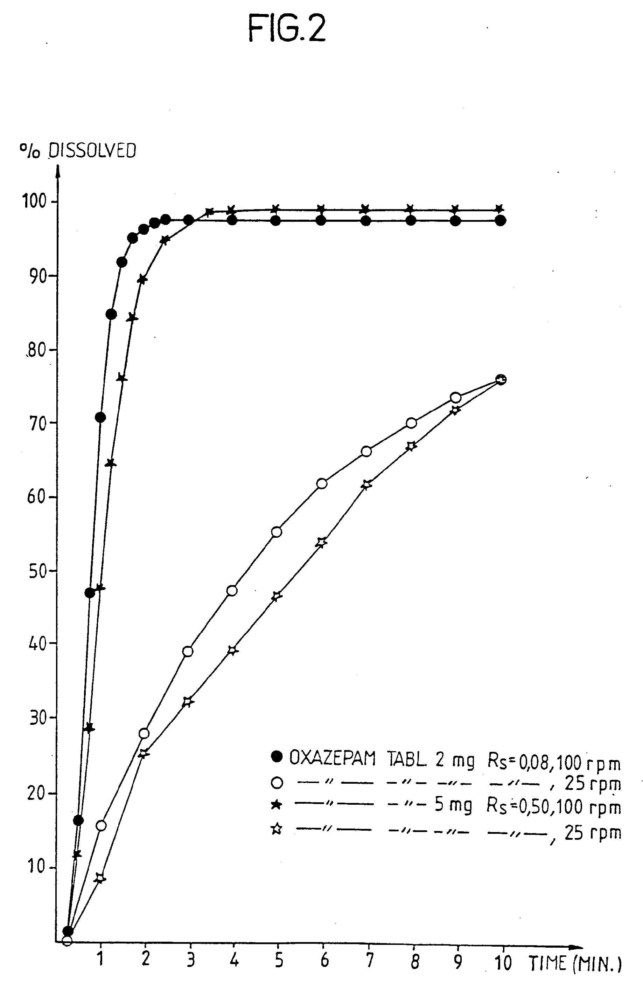

The dissolution rate of the tablets thus produced was investigated in accordance with U.S.P. XXI, the paddle method, at two mutually different stirring speeds, 100 and 25 rpm. Tablets produced in accordance with Example 1 above were also investigated, for comparison purposes. Each of these latter tablets contained 2 mg of oxazepam and exhibited a surface area ratio of 0.08. The tablets had been compacted at a pressure of 200 MPa. The results of these investigations are presented in Figure 2. It will clearly be seen from this figure that the dissolution rate of an ordered mixture according to the invention with each tablet containing 5 mg of oxazepam and exhibiting a surface area ratio of 0.5 is essentially the same as that of the prior known tablets each containing 2 mg of oxazepam and exhibiting a surface area ratio of 0.08. These results were obtained at both of the stirring speeds applied.

### Example 3

It is known to the art that an addition of an hydrophobic lubricant, such as magnesium stearate, can have a negative effect on the rate at which a tablet will dissolve. It is also known that this negative effect will increase with greater quantities of lubricant and longer admixing times.

This example illustrates how the addition of a lubricant affects the dissolution rate of a tablet which contains an ordered mixture. Two types of carrier substance were investigated. The substances or materials used were mannitol, which will fragmentize, and sodium chloride, which will not fragmentize.

Each of the substances mannitol and sodium chloride, with a particle size of 250-450 µm, were dry mixed with oxazepam, which had an external specific surface area of not less than 20000 cm²/g, such as to obtain the surface area ratios of 0.51 and 0.41, which corresponded to an oxazepam addition of 1.3% w/w and 0.58% w/w respectively. The dissolution rate of each of the mixtures was then investigated in accordance with U.S.P. XXI, the paddle method, at room temperature and a stirring speed of 100 rpm.

Tablets were then produced from each of the ordered mixtures, by mixing these mixtures with 5% w/w Avicel ^{R} PH 101 and 2% w/w Ac-Di-Sol ^{R} for 30 mins. The ordered mixture that contained mannitol as the carrier substance was divided into two parts. 0.2% w/w magnesium stearate was added to one part and 1% w/w magnesium stearate to the other. 1% w/w magnesium stearate was added to the mixture containing sodium chloride. The three mixtures were re-mixed for 10 mins. The resultant mixtures were compacted into tablet form, each containing 2 mg of oxazepam, at a pressure of 200 MPa. The dissolution rate of these tablets was investigated in accordance with U.S.P. XXI, the paddle method, at room temperature and a stirring speed of 100 rpm.

The results of these investigations are presented in Figure 3. The figure shows that a drug which comprises an ordered mixture with mannitol as the carrier substance will dissolve very quickly. An addition of 0.2% w/w magnesium prior to compacting the mixture does not affect the dissolution rate of the tablet. This is assumed to be because the carrier substance fragmentizes to expose fresh surfaces which facilitate access of the solvent to the carrier. The drug does not dissolve quite so well when the amount of magnesium stearate included is increased to 1% w/w. This is assumed to be because the increase in lubricant has a more negative influence on the dissolution rate, possibly due to a lesser degree of fragmentization of mannitol.

A drug which is composed of an ordered mixture with sodium chloride as the carrier substance will also dissolve quickly. The dissolution rate is impaired very considerably, when 1% w/w magnesium stearate is added prior to compaction. This is presumed to be because sodium chloride is not fragmentized during compaction, therewith rendering access of the solvent to the carrier difficult.

The investigation showed that in order to avoid negative effects on the dissolution rate of a drug, a fragmentizing carrier substance should be used when a lubricant is added.

It was also found that when practising the present invention a tablet could contain twice as much pharmaceutically active substance as a tablet formulated in accordance with known techniques, while still obtaining an equally rapid dissolution rate, which is a requirement for obtaining an equally rapid effect of the drug upon administration. Thus, the possibility of being able to increase the dosage while retaining quick release of the drug is highly significant, since the majority of pharmaceutical preparations contain dosages which exceed the maximum quantity supplied with known techniques. The important advantages afforded hereby in drug administration will be readily perceived.

In the aforegoing examples, the invention has been described primarily with reference to the use of a specific combination of carrier and active substance. It will be apparent to those skilled in this art, however, that the invention is not limited to this combination and that each other combination of carrier and active substance is also possible within the scope of the claims.

## Claims (Claims for the following Contracting State(s): AT, BE, CH, DE, FR, GB, IT, LI, LU, NL,SE)

1. An ordered mixture of particles of a water-soluble, pharmaceutically acceptable carrier substance which has adhering thereto smaller particles of at least one pharmaceutically active substance, characterized in that the carrier substance is a substance which will fragmentize heavily when compressed, and that the particles of carrier substance have a pharmaceutical disintegrant incorporated therein.

2. An ordered mixture according to Claim 1, characterized in that the particles of carrier substance contain up to 25 % by weight, and preferably up to 10 % by weight of disintegrant.

3. An ordered mixture according to Claim 1 or 2, characterized in that the particles of carrier substance have a size from 50 to 1000 µm, and then preferably from 100 to 500 µm.

4. An ordered mixture according to any of Claims 1-3, characterized in that the particles of pharmaceutically active substance have a size of at most 25 µm and then preferably at most 10 µm.

5. An ordered mixture according to any of Claims 1-4, characterized in that the carrier substance is a water-soluble, pharmaceutically acceptable carbohydrate or inorganic salt.

6. An ordered mixture according to Claim 5, characterized in that the carrier substance is mannitol or lactose.

7. An ordered mixture according to any of Claims 1-6, characterized in that the pharmaceutically active substance or substances is, or are, bound to the carrier particles in an amount which corresponds to a surface area ratio of at most about 0.5.

8. An ordered mixture according to any of Claims 1-7, characterized in that the carrier particles also have adhering thereto small particles of the same or a different pharmaceutical, acceptable carrier substance having a particle size in the same order of magnitude as the particles of active substance and in an amount of up to 100 % by weight thereof.

9. A pharmaceutical preparation, characterized in that it includes an ordered mixture according to any one of Claims 1-8, together with pharmaceutically acceptable additives and excipients.

10. A pharmaceutical preparation according to Claim 9, characterized in that it has a substantially water-free form for internal use.

11. A method of producing a pharmaceutical preparation according to Claim 9 or Claim 10, characterized by combining an ordered mixture according to any of Claims 1-8 with pharmaceutically acceptable additives and excipients.

12. The use of an ordered mixture according to any of Claims 1-8 when producing a pharmaceutical preparation according to Claim 9 or Claim 10.

## Claims (Claims for the following Contracting State(s): ES, GR)

1. A method for the preparation of an ordered mixture of particles of a water-soluble, pharmaceutically acceptable carrier which has adhering thereto smaller particles of at least one pharmaceutically active substance, characterized in that in a carrier substance which will fragmentize heavily when compressed is incorporated a pharmaceutical disintegrant to form a mixture which is shaped to granules, after which at least one pharmaceutically active substance is adhered to said granules.

2. A method according to claim 1, characterized in that up to 25 % by weight, and preferably up to 10 % by weight of the disintegrant is incorporated into the carrier granules.

3. A method according to claim 1 or 2, characterized in that the granules of the carrier have a size from 50 to 1000 µm, and preferably then from 100 to 500 µm.

4. A method according to any of claims 1-3, characterized in that the pharmaceutically active substance or substances which is, or are, adhered to the carrier granules has a particle size of at most 25 µm, and preferably then at most 10 µm.

5. A method according to any of claims 1-4, characterized in that the carrier substance is a water-soluble , pharmaceutically acceptable carbohydrate or inorganic salt.

6. A method according to claim 5, characterized in that the carrier substance is mannitol or lactose.

7. A method according to any of claims 1-6, characterized in that the pharmaceutically active substance or substances is, or are, adhered to the carrier granules in an amount which corresponds to a surface area ratio of at most about 0.5.

8. A method according to any of claims 1-7, characterized in that to the carrier granules are also adhered small particles of the same or a different pharmaceutically acceptable carrier substance, which has a particle size in the same order of magnitude as the particles of the pharmaceutically active substance, in an amount of up to 100 % by weight thereof.

9. A method according to any of claims 1-8, characterized in that the ordered mixture prepared is incorporated in a pharmaceutical preparation, together with pharmaceutically acceptable additives and excipients.

10. A method according to claim 9, characterized in that the additives and excipients are selected such that a substantially water-free form for internal use is obtained.

## Patentansprüche (Patentansprüche für folgende(n) Vertragsstaat(en): AT, BE, CH, DE, FR, GB, IT, LI, LU, NL, SE)

1. Geordnetes Gemisch von Teilchen einer wasserlöslichen, pharmazeutisch verträglichen Trägersubstanz, an der kleinere Teilchen von mindestens einer pharmazeutisch wirksamen Substanz haften, dadurch gekennzeichnet, daß die Trägersubstanz eine Substanz ist, die beim Komprimieren schwer fragmentiert, und daß in den Teilchen der Trägersubstanz ein pharmazeutisches Sprengmittel enthalten ist.

2. Geordnetes Gemisch nach Anspruch 1, dadurch gekennzeichnet, daß die Teilchen der Trägersubstanz bis zu 25 Gew.-%, vorzugsweise bis zu 10 Gew.-%, des Sprengmittels enthalten.

3. Geordnetes Gemisch nach Anspruch 1 oder 2, dadurch gekennzeichnet, daß die Teilchen der Trägersubstanz eine Größe von 50 bis 1000 µm, vorzugsweise von 100 bis 500 µm, aufweisen.

4. Geordnetes Gemisch nach einem jeden der Ansprüche 1 bis 3, dadurch gekennzeichnet, daß die Teilchen der pharmazeutisch wirksamen Substanz eine Größe von höchstens 25 µm, vorzugsweise von höchstens 10 µm, aufweisen.

5. Geordnetes Gemisch nach einem jeden der Ansprüche 1 bis 4, dadurch gekennzeichnet, daß die Trägersubstanz ein wasserlösliches, pharmazeutisch verträgliches Kohlehydrat oder anorganisches Salz ist.

6. Geordnetes Gemisch nach Anspruch 5, dadurch gekennzeichnet, daß die Trägersubstanz Mannitol oder Lactose ist.

7. Geordnetes Gemisch nach einem jeden der Ansprüche 1 bis 6, dadurch gekennzeichnet, daß die pharmazeutisch wirksame Substanz bzw. die Substanzen an die Trägerteilchen in einer Menge entsprechend einem Oberflächenverhältnis von höchstens etwa 0,5 gebunden ist bzw. sind.

8. Geordnetes Gemisch nach einem jeden der Ansprüche 1 bis 7, dadurch gekennzeichnet, daß die Trägerteilchen weiterhin an ihnen anhaftende kleine Teilchen der gleichen oder einer anderen pharmazeutisch verträglichen Trägersubstanz mit einer Teilchengröße in der gleichen Größenordnung wie die Teilchen der Wirksubstanz und in einer Menge von bis zu 100 Gew.-% derselben aufweisen.

9. Pharmazeutische Zubereitung, dadurch gekennzeichnet, daß sie ein geordnetes Gemisch gemäß einem jeden der Ansprüche 1 bis 8 zusammen mit pharmazeutisch verträglichen Additiven und Exzipienten enthält.

10. Pharmazeutische Zubereitung gemäß Anspruch 9, dadurch gekennzeichnet, daß sie eine im wesentlichen wasserfreie Form zur internen Verwendung aufweist.

11. Verfahren zur Herstellung einer pharmazeutischen Zubereitung gemäß Anspruch 9 oder Anspruch 10, dadurch gekennzeichnet, daß man ein geordnetes Gemisch gemäß einem jeden der Ansprüche 1 bis 8 mit pharmazeutisch verträglichen Additiven und Exzipienten kombiniert.

12. Verwendung eines geordneten Gemischs gemäß einem jeden der Ansprüche 1 bis 8 bei der Herstellung einer pharmazeutischen Zubereitung gemäß Anspruch 9 oder Anspruch 10.

## Patentansprüche (Patentansprüche für folgende(n) Vertragsstaat(en): ES, GR)

1. Verfahren zur Herstellung eines geordneten Gemischs von Teilchen einer wasserlöslichen, pharmazeutisch verträglichen Trägersubstanz, an der kleinere Teilchen von mindestens einer pharmazeutisch wirksamen Substanz haften, dadurch gekennzeichnet, daß einer Trägersubstanz, die beim Komprimieren schwer fragmentiert, ein pharmazeutisches Sprengmittel unter Ausbildung eines Gemischs, das zu Körnchen geformt wird, zugesetzt wird, wonach mindestens eine pharmazeutisch wirksame Substanz an die Körnchen angeheftet wird.

2. Verfahren nach Anspruch 1, dadurch gekennzeichnet, daß bis zu 25 Gew.-%, vorzugsweise bis zu 10 Gew.-%, des Sprengmittels den Trägerkörnchen einverleibt wird.

3. Verfahren nach Anspruch 1 oder 2, dadurch gekennzeichnet, daß die Körnchen des Trägermaterials eine Größe von 50 bis 1000 µm, vorzugsweise von 100 bis 500 µm, aufweisen.

4. Verfahren gemäß einem jeden der Ansprüche 1 bis 3, dadurch gekennzeichnet, daß die pharmazeutisch wirksame Substanz bzw. Substanzen, die an den Trägerkörnchen anhaftet bzw. anhaften, eine Teilchengröße von höchstens 25 µm, vorzugsweise von höchstens 10 µm, aufweist bzw. aufweisen.

5. Verfahren gemäß einem jeden der Ansprüche 1 bis 4, dadurch gekennzeichnet, daß die Trägersubstanz ein wasserlösliches, pharmazeutisch verträgliches Kohlehydrat oder anorganisches Salz ist.

6. Verfahren gemäß Anspruch 5, dadurch gekennzeichnet, daß die Trägersubstanz Mannitol oder Lactose ist.

7. Verfahren gemäß einem jeden der Ansprüche 1 bis 6, dadurch gekennzeichnet, daß die pharmazeutisch wirksame Substanz bzw. Substanzen an die Trägerkörnchen in einer Menge entsprechend einem Oberflächenverhältnis von höchstens etwa 0,5 angeheftet wird bzw. werden.

8. Verfahren gemäß einem jeden der Ansprüche 1 bis 7, dadurch gekennzeichnet, daß an die Trägerteilchen weiterhin kleine Teilchen der gleichen oder einer anderen, pharmazeutisch verträglichen Trägersubstanz angeheftet werden, die eine Teilchengröße in der gleichen Größenordnung wie die Teilchen der pharmazeutisch wirksamen Substanz aufweisen, und zwar in einer Menge von bis zu 100 Gew.-% derselben.

9. Verfahren gemäß einem jeden der Ansprüche 1 bis 8, dadurch gekennzeichnet, daß das hergestellte, geordnete Gemisch einer pharmazeutischen Zubereitung zusammen mit pharmazeutisch verträglichen Additiven und Exzipienten zugesetzt wird.

10. Verfahren gemäß Anspruch 9, dadurch gekennzeichnet, daß die Additive und Exzipienten so ausgewählt sind, daß eine im wesentlichen wasserfreie Form zur internen Verwendung erhalten wird.

## Revendications (Revendications pour l'(les) Etat(s) contractant(s) suivant(s): AT, BE, CH, DE, FR, GB, IT, LI, LU, NL, SE)

1. Mélange ordonné de particules d'une substance de véhicule hydrosoluble pharmaceutiquement compatible, substance sur laquelle adhèrent de plus petites particules d'au moins une substance pharmaceutiquement active, caractérisé en ce que la substance véhicule est une substance qui se fragmente fortement à l'état comprimé et en ce que les particules de la substance véhicule comportent un désagrégeant pharmaceutique incorporé dans celle-ci.

2. Mélange ordonné selon la revendication 1, caractérisé en ce que les particules de la substance véhicule contiennent jusqu'à 25 % en poids, et de préférence jusqu'à 10 % en poids de désagrégeant.

3. Mélange ordonné selon la revendication 1 ou 2, caractérisé en ce que les particules de la substance véhicule ont une granulométrie de 50 à 1000 µm, et de préférence de 100 à 500 µm.

4. Mélange ordonné selon l'une quelconque des revendications 1-3, caractérisé en ce que les particules de la substance pharmaceutiquement active ont une granulométrie de 25 µm au plus et de préférence de 10 µm au plus.

5. Mélange ordonné selon l'une quelconque des revendications 1-4, caractérisé en ce que la substance véhicule est un hydrate de carbone hydrosoluble pharmaceutiquement compatible ou un sel inorganique.

6. Mélange ordonné selon la revendication 5, caractérisé en ce que la substance véhicule est du mannitol ou du lactose.

7. Mélange ordonné selon l'une quelconque des revendications 1-6, caractérisé en ce que la (ou les) substance(s) active(s) pharmaceutiquement est (ou sont) liée(s) aux particules véhicules dans une quantité qui correspond à un rapport d'aire de surface d'environ 0,5 au plus.

8. Mélange ordonné selon l'une quelconque des revendications 1-7, caractérisé en ce que les particules véhicules comportent également des petites particules de la mêmè substance véhicule, pharmaceutiquement compatible, ou d'une substance véhicule pharmaceutiquement compatible différente ayant une granulométrie dans le même ordre de grandeur que les particules de la substance active et dans une quantité jusqu'à 100 % en poids.

9. Préparation pharmaceutique, caractérisée en ce qu'elle comprend un mélange ordonné selon l'une quelconque des revendications 1-8, conjointement avec des excipients et additifs pharmaceutiquement compatibles.

10. Préparation pharmaceutique selon la revendication 9, caractérisée en ce qu'elle possède une forme sensiblement exempte d'eau pour usage interne.

11. Procédé pour la production d'une préparation pharmaceutique selon la revendication 9 ou la revendication 10, caractérisé par la combinaison d'un mélange ordonné selon l'une quelconque des revendications 1-8 avec des excipients et additifs pharmaceutiquement compatibles.

12. Utilisation d'un mélange ordonné selon l'une quelconque des revendications 1-8 lors de la production d'une préparation pharmaceutique selon la revendication 9 ou la revendication 10.

## Revendications (Revendications pour l'(les) Etat(s) contractant(s) suivant(s): ES, GR)

1. Procédé pour la préparation d'un mélange ordonné de particules d'un véhicule hydrosoluble pharmaceutiquement compatible qui comporte des particules plus petites adhérant sur celui-ci d'au moins une substance active pharmaceutiquement, caractérisé en ce que dans une substance véhicule qui se fragmente fortement à l'état comprimé, on incorpore un désagrégeant pharmaceutique pour former un mélange sous forme de granules après quoi au moins une substance active pharmaceutiquement adhère sur ces granules.

2. Procédé selon la revendication 1, caractérisé en ce que l'on incorpore jusqu'à 25 % en poids, de préférence jusqu'à 10 % en poids du désagrégeant dans les granules véhicules.

3. Procédé selon la revendication 1 ou 2, caractérisé en ce que les granules du véhicule ont une granulométrie de 50 à 1000 µm et de préférence de 100 à 500 µm.

4. Procédé selon l'une quelconque des revendications 1-3, caractérisé en ce que la (ou les) substance(s) active(s) pharmaceutiquement qui adhère(nt) sur les granules véhicules a (ou ont) une granulométrie de 25 µm au plus et de préférence de 10 µm au plus.

5. Procédé selon l'une quelconque des revendications 1-4, caractérisé en ce que la substance véhicule est un hydrate de carbone hydrosoluble, pharmaceutiquement compatible ou un sel minéral.

6. Procédé selon la revendication 6, caractérisé en ce que la substance véhicule est du mannitol ou du lactose.

7. Procédé selon l'une quelconque des revendications 1-6, caractérisé en ce que la (ou les) substance(s) active(s) pharmaceutiquement, adhère(nt) aux granules véhicules dans une quantité qui correspond à un rapport d'aire de surface d'environ 0,5 au plus.

8. Procédé selon l'une quelconque des revendications 1-7, caractérisé en ce que sur les granules véhicules adhèrent également des petites particules de la même substance véhicule pharmaceutiquement compatible ou d'une substance véhicule pharmaceutiquement compatible différente ayant une granulométrie dans le même ordre de grandeur que les particules de la substance active pharmaceutiquement, dans une quantité jusqu'à 100 % en poids.

9. Procédé selon l'une quelconque des revendications 1-8, caractérisé en ce que le mélange ordonné préparé est incorporé dans une préparation pharmaceutique conjointement avec des additifs et excipients pharmaceutiquement compatibles.

10. Procédé selon la revendication 9, caractérisé en ce que les additifs et excipients sont choisis de façon à obtenir une forme sensiblement exempte d'eau pour usage interne.
